# EUROPEAN PATENT SPECIFICATION

(11) **EP 4 129 367 B1**
(45) Date of publication and mention of the grant of the patent: **03.12.2025**
(21) Application number: 21189347.4
(22) Date of filing: 03.08.2021
(51) Int. Cl.: A61M 5/32

(54) **CAP ASSEMBLY FOR A NEEDLE**
KAPPENANORDNUNG FÜR EINE NADEL
ENSEMBLE CAPUCHON POUR UNE AIGUILLE

(43) Date of publication of application: 08.02.2023
(73) Proprietor: Gerresheimer Regensburg GmbH, 93047 Regensburg (DE)
(72) Inventor: Fraas, Andreas, 92224 Amberg (DE); Mehlhaf, Eduard, 92256 Hahnbach (DE)
(74) Representative: Fish & Richardson P.C.

(56) References cited:
- EP-A1- 3 881 881
- US-A1- 2018 326 161
- US-A1- 2020 179 614
- US-B2- 10 537 688

## Description

This disclosure relates to a cap assembly for enclosing a needle of a needle device and to a needle device including such a cap assembly.

Hollow needles, in particular hypodermic needles, can be used in a variety of contexts. For example, a hypodermic needle is used to inject various pharmaceutical, cosmetic, or veterinary preparations. A hypodermic needle can also be used to draw blood. Hypodermic needles can be used for syringes, auto-injectors, pen injectors, and winged infusion sets for venipuncture, to name a few examples. Since hypodermic needles are designed to pierce skin, they are sometimes a source of needlestick injuries, i.e., wounds that arise when the skin is accidentally punctured by a needle. Some blood borne infections and diseases can be transmitted when a hypodermic needle is re-used. A cap can be used to enclose the needle of a needle device and prevent such issues.

US 2020/179614 A1 describes a syringe safety system that may include a sleeve. The sleeve may include a guide track formed in the sleeve. The guide track may have a guide pin retention portion, a travel portion, and a lockout portion. The syringe safety system may include a collar having a guide from an exterior surface of the collar. A spring may extend between and be coupled to the collar and the sleeve. A cap may engage the sleeve. The cap may be configured to retain the guide pin in the guide pin retention portion of the guide tack. When the cap is removed, the spring advances the guide pin into the travel portion of the guide track.

US 2018/326161 A1 describes a needle safety device that has an outer tube within which a syringe barrel from which a cannula extends is slideably receivable. A collar in the outer tube is moveable relative thereto and rotatably attachable to the distal end of the barrel. A force member biases the outer tube in a distal direction. A cannula shield able to receive the cannula therein is in the outer tube. The cannula shield is fixedly attached to a cap removably attached to the outer tube. A track is formed in the inner surface of the outer tube. A pin extending radially outwardly from the collar slidingly engages the track. In a pre-injection position, the cannula is entirely within the outer tube. In a full-insertion position, the cannula extends beyond the outer tube. In a locked position, the cannula is irreversibly retained entirely within the outer tube.

US 10 537 688 B2 describes a syringe member comprising a safety device to prevent pricking injuries; the syringe member comprises a pricking element, and the safety device includes at least one guided pin and a recess which forms a guide slot for guiding the guided pin in a longitudinal direction of the syringe member during movement of the syringe member relative to the safety device; said guide slot includes a first slot region and a second slot region which are separated by a notional separation line extending in a longitudinal direction of the syringe member; the guided pin can be placed in the first slot region in a starting position and can be moved from the first slot region into the second slot region into a final position by having the guided pin cross the separation line when a distal end of the pricking element is located at the level of an exit opening of the safety device.

EP 3 881 881 A1 describes a safety device for injection devices. The safety device includes a sleeve extending along a longitudinal axis of the injection device's body, at least partially enclosing the injection device's needle and body, and including a guide slot; a collar attached to a distal end region of the body and locking the safety device in an axial direction; and a cap that can be arranged at least in part over the sleeve to prevent the body from moving relative to the sleeve. The cap includes a receptacle for the needle. The collar includes a guide pin that engages the guide slot. The collar can be arranged to rotate relative to the sleeve, and the receptacle of the cap can engage the collar and prevent the collar from rotating. The sleeve and the collar can each include a lock.

According to the present invention, a cap assembly for enclosing a needle of a needle device includes a sleeve and a collar. The sleeve extends along a longitudinal axis and defines a first opening configured to receive the needle device, a second opening opposite to the first opening and sized such that the needle device's needle can protrude through the second opening, and a slot that extends substantially along the longitudinal axis. The collar is arranged in the sleeve and biased away from the second opening of the sleeve. The collar is configured to translate along the longitudinal axis of the sleeve and includes a first collar section that defines a pin that protrudes through the sleeve's slot, and a second collar section, wherein the first and second collar section cooperate to define a collar body configured to engage the needle device.

The first collar section and the second collar section can each define an opening that extends in parallel to the longitudinal axis and is configured to receive the needle device.

The first collar section and the second collar section can be configured to abut along two or more surfaces that extend substantially radially to and along the longitudinal axis.

The first collar section and the second collar section can be configured to interlock to prevent relative movement of the first and second collar sections along or relative to the longitudinal axis. For example, the first collar section and the second collar section can be configured for snap-fit engagement.

In some cases, the first collar section encloses the longitudinal axis of the sleeve.

The slot can include a first closed end adjacent to the first opening and a second closed end adjacent to the second opening. The first closed end can include a retaining hook configured to engage the pin of the first collar section.

The first collar section and the second collar section are configured to rotate relative to one another about the longitudinal axis. For example, the first and the second collar sections form a ratchet that allows rotation about the longitudinal axis in only one direction. The first and the second collar sections can optionally include a plurality of teeth that extend in parallel to the longitudinal axis and mate to form the ratchet. In some cases, the second collar section can define a pin that protrudes through a further slot defined by the sleeve and extending substantially along the longitudinal axis. For example, the slot that receives the pin of the first collar section can extend in parallel to the longitudinal axis, and the slot that receives the pin of the second collar section can include a straight portion that extends in parallel to the longitudinal axis and a branched portion that extends laterally to the longitudinal axis.

In some embodiments, the collar consists of the first collar section and the second collar section. In other embodiments, the collar can include further components in addition to the first collar section and the second collar section.

According to the present invention, an assembly includes a cap assembly as previously described and an needle device that includes a body configured to receive a liquid, a hollow needle that communicates with an interior of the body, and a tip that connects the needle and the body and defines a portion having an increased diameter. The collar body includes an end surface configured to abut the increased diameter portion of the tip of the needle device.

According to the present invention, an assembly includes a cap assembly as previously described and an needle device that includes a body configured to receive a liquid, a hollow needle that communicates with an interior of the body, and a tip that connects the needle and the body and defines a groove that encircles the longitudinal axis. The collar body includes a projection received in the groove of the needle device's tip.

In all of the aspects, the needle device may be a syringe, an auto-injector, or a pen injector to name a few examples.

These and other embodiments described herein may provide one or more of the following benefits. The two-part collar described in the embodiments may be easy to assemble in comparison to other devices. For instance, the collar may require little to no modification of the needle device to which it is attached. In some embodiments, the assembly may include features that prevent reuse of the needle.

Certain embodiments will now be described, by way of example only, with reference to the accompanying drawings, in which:
Figure 1 is a schematic partial depiction of a needle device;
Figures 2A and 2B show a schematic cross-section of the needle device of Figure 1 with an example cap assembly;
Figures 3A and 3B are side views of Figures 2A and 2B;
Figures 4A to 4D are perspective views of a non-claimed example of a collar;
Figures 5A to 5D are perspective views of a further example collar 100;
Figures 6A and 6B schematically depict the collar of Figures 5A to 5D from above;
Figures 6C to 6E depict an example cap assembly that includes the collar of Figures 5A to 6B;
Figures 7A to 7C depict an assembly that is similar to the assembly 300 of Figures 6C to 6E; and
Figures 8A to 8D are perspective views of an example collar; and
Figure 9 is a schematic partial depiction of a further needle device.

Like reference numbers and designations in the various drawings indicate like elements.

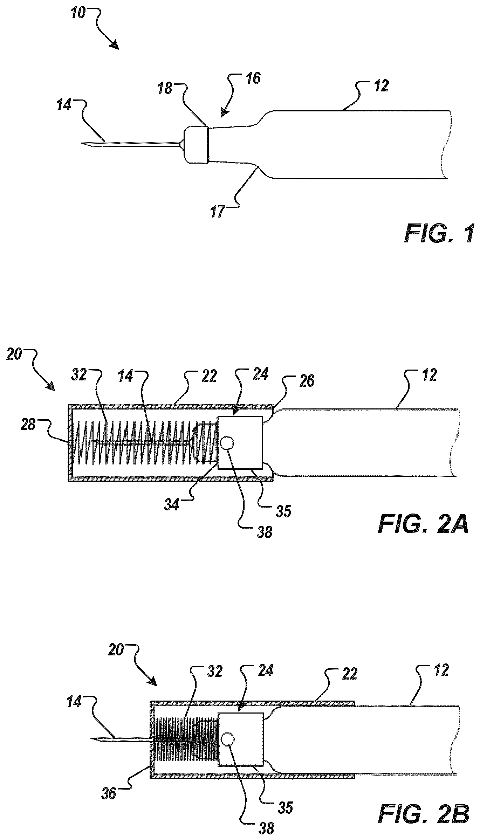

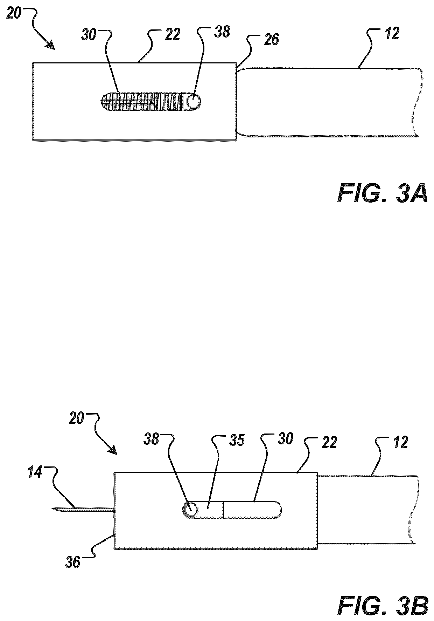

Figure 1 is a schematic partial depiction of a needle device 10. The needle device 10 includes a body 12 that is configured to receive a liquid, a hollow needle 14 (e.g., a hypodermic needle) that communicates with an interior of the body 12, and a tip 16 that connects the needle 14 and the body 12. The needle device 10 can include a rounded shoulder 17 that connects the body 12 and the tip 16. For example, the needle device 10 may be a syringe, an auto-injector, or a pen injector for hypodermically administrating medications. Since the needle 14 is designed to pierce skin, the needle 14 can lead to needlestick injuries when handled by a user. Accordingly, a cap assembly can be provided to enclose the needle 14 and prevent such injuries, as is described in more detail in reference to Figures 2 and 3.

Figures 2A and 2B show a schematic cross-section of the needle device 10 of Figure 1 with an example cap assembly 20. Figures 3A and 3B are side views of Figures 2A and 2B. The cap assembly 20 encloses the needle 14 when the needle device 10 is not in use (Figure 2A). The cap assembly includes a sleeve 22 and a collar 24. In some instances, the cap assembly 20 can include further components that are omitted from Figures 2 and 3 for the sake of simplicity.

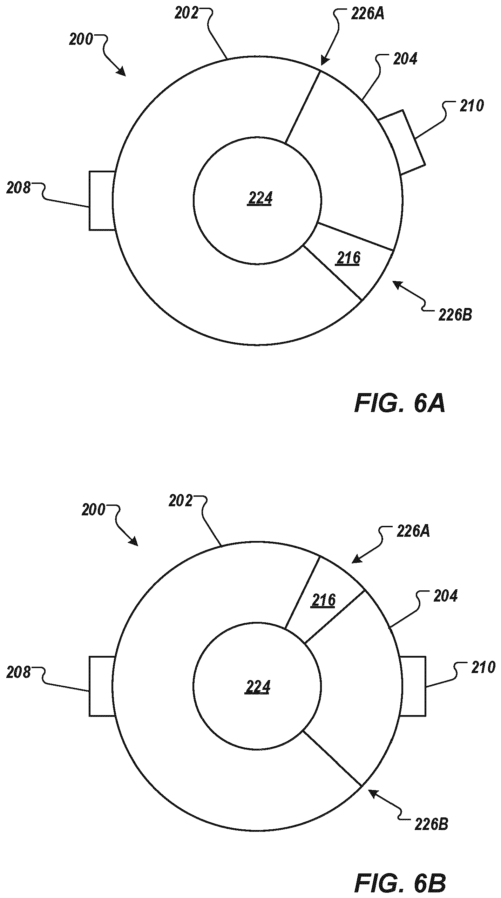

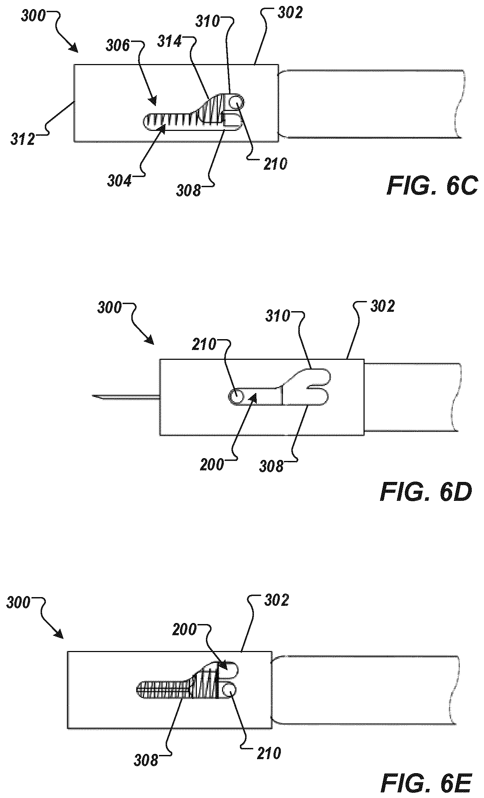

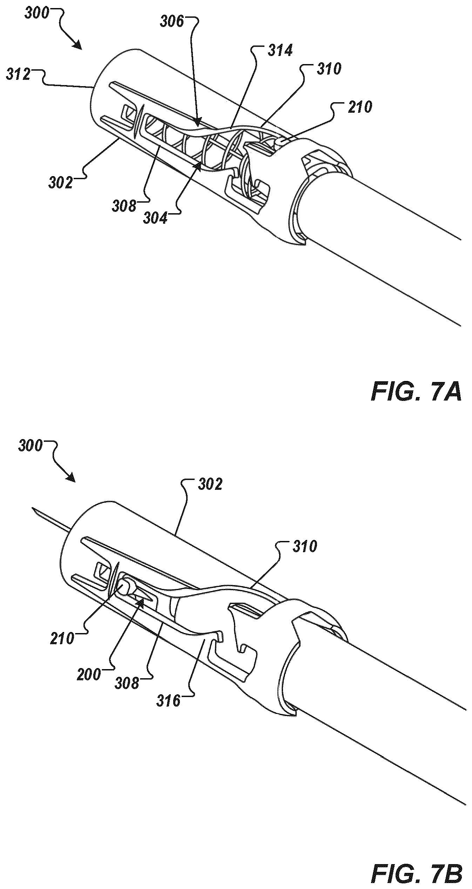

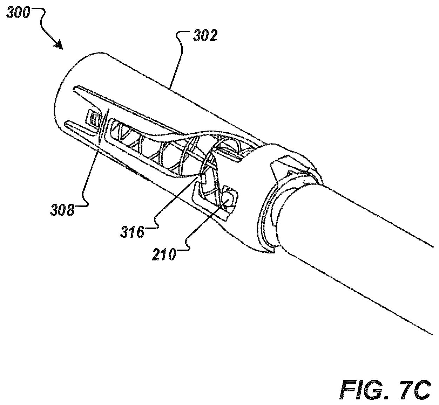

As shown in Figure 2A, the sleeve 22 extends along a longitudinal axis and defines a first opening 26 and a second opening 28. The first opening 26 is configured to receive the needle device 10. The second opening 28 is sized such that the device's needle 14 can protrude through the second opening 28 (Figure 2B). For example, the second opening 28 can be smaller than the first opening 26. However, in some instances, the second opening 28 can also be the same size as the first opening 26. As shown in Figures 3A and 3B, the sleeve 22 also includes a slot 30 that extends substantially along the longitudinal axis. In some cases, the sleeve 22 includes two slots, e.g., on one on each side of the sleeve 22. Instead of two slots and pins, the collar may include, e.g., a projection that slides along a corresponding ramp provided in the sleeve 22. Although the slot 30 is depicted as a substantially straight slot 30 that extends along the sleeve's longitudinal axis, other slot shapes are shown in Figures 6 and 7, for example.

The collar 24 is arranged in the sleeve 22 and biased away from the second opening 28 of the sleeve 22, e.g., by a spring 32. For example, the spring 32 cooperates with a first end surface 34 of the collar 24 to bias the collar 24 towards the first opening 26 of the sleeve 22 (Figures 2A and 3A). Generally, the collar 24 includes a collar body 35 configured to engage the needle device 10. For example, in Figures 1 to 3, the tip 16 of the needle device 10 defines a ridge 18 that is configured to abut the first end surface 34 of the collar body 35. Thus, when the spring 32 biases the collar 24 towards the first opening 26 of the sleeve 22, the needle device 10 is also moved towards the first opening 26. In this position, the sleeve 22 fully enclosed the needle 14 and protects the user from potential needlestick injuries. The collar 24, the sleeve 22, and the needle device 10 are arranged coaxially, such that in the following, reference is made to each of their respective longitudinal axes interchangeably.

As shown in Figures 2B and 3B, the spring 32 can be compressed to translate the collar 24 along the longitudinal axis of the sleeve 22. In other words, the collar 24 moves closer towards the second opening 28 of the sleeve 22, which causes the needle 14 to extend through the second opening 28. In this position, the needle device 10 can be used to hypodermically administer a medication, for example. The sleeve 22 can be retracted by gripping an outer surface of the sleeve 22 or by placing an end surface 36 of the sleeve 22 against a patient's skin and applying pressure to the body 12 of the needle device 10. Once the injection has been administered, the user can release the sleeve 22, which returns the sleeve to the position shown in Figures 2A and 3A.

Generally speaking, the collar body 35 defines a pin 38 that protrudes through the slot 30 in the sleeve (Figures 3A and 3B). In embodiments in which the sleeve 22 includes more than one slot 30, the collar body 35 may define a pin 38 for each slot 30. The slot 30 and pin 38 can cooperate to control the movement of the collar 24 and the needle device 10 as the spring 32 expands and contracts.

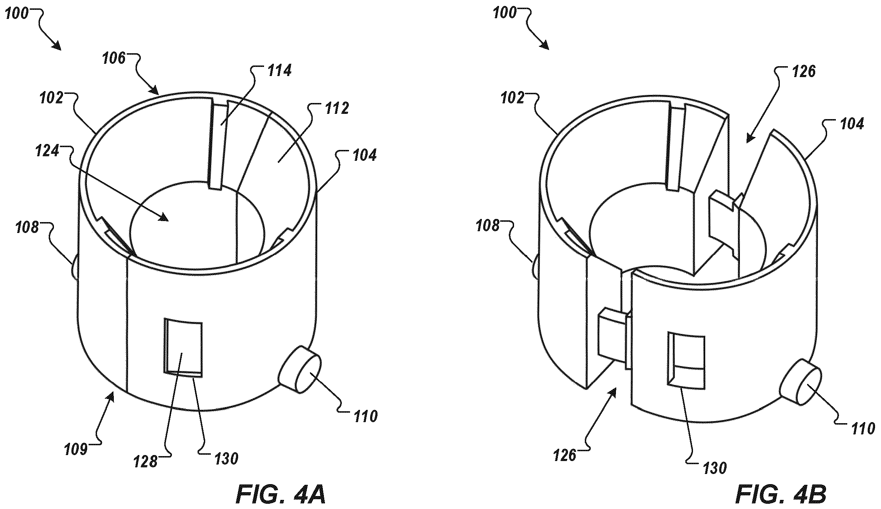

Figures 4A to 4D are perspective views of a non-claimed example of a collar 100. The collar 100 includes a first collar section 102 and a second collar section 104 that cooperate to define a collar body 106 configured to engage a needle device. For example, the collar 100 can correspond to the collar 24 shown in Figures 2 and 3 and engage the needle device 10 of Figure 1.

For example, an end surface 109 of the collar body 106 can be configured to engage the ridge 18 defined by the tip 16 of the needle device 10. Opposite the end surface 109, the collar body 106 can include an angled surface 112 that is configured to face a shoulder of the needle device, e.g., the shoulder 17 of needle device 10 in Figure 1. In some instances, the entire angled surface 112 is designed to contact the shoulder of the needle device. In some embodiments, the angled surface 112 includes protruding ribs 114 that lie against the surface of the shoulder.

Although the device 10 of Figure 1 is shown having a ridge 18 that engages the end surface 109 of the collar body 106, this is not necessarily the case. Instead of a pronounced ridge 18, the tip 16 may merely have a larger diameter between the collar body 106 and the needle 14, and the collar body 106 may abut the increased diameter portion. The transition between the increased diameter portion and the remainder of the tip 16 may be rounded, such that the collar body 106 is arranged on a narrowed section of the tip 16 between the shoulder 17 and the needle 14.

The collar body 106 can have a generally cylindrical shape, as shown in Figures 4A and 4B. The first collar section 102 defines a pin 108 that is configured to protrude through a slot in a sleeve, e.g., the slot 30 in sleeve 22. As illustrated, the first and second collar sections 102, 104 each define a pin 108, 110. In other embodiments, the collar body 106 can have a non-cylindrical shape (e.g., a hexagonal shape).

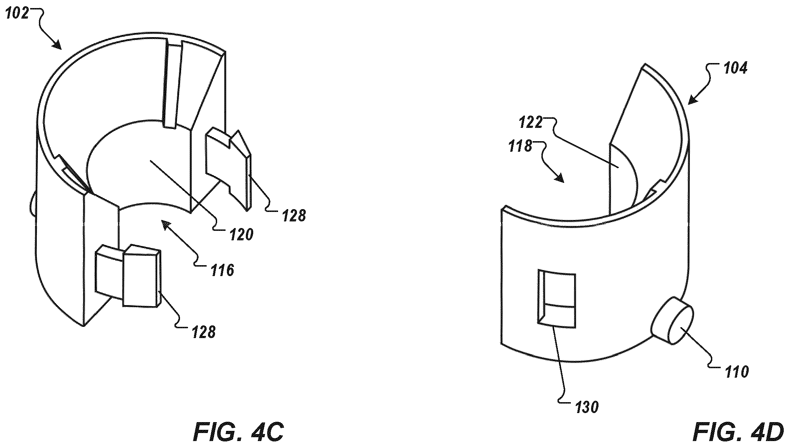

As shown in Figures 4C and 4D, the first collar section 102 and the second collar section 104 each define an opening 116, 118 that extends in parallel to the longitudinal axis and is configured to receive the needle device. For example, the openings 116, 118 are each bounded by a semi-cylindrical surface 120, 122. The first and second collar sections 102, 104 each form a shell that extends about the longitudinal axis of the collar body without enclosing said axis. The semi-cylindrical surfaces 120, 122 cooperate to define a smooth cylindrical throughbore 124 (Figure 4A) in the collar body 106 that allows the collar 100 to rotate about the needle device. However, in other embodiments, the opening can have a different shape.

As described, the first collar section 102 and the second collar section 104 cooperate to form the collar body 106. For example, the first and second collar sections 102, 104 are configured to abut along two pairs of mating surfaces 126 shown in Figure 4B. For example, the mating surfaces 126 can extend substantially radially relative to a longitudinal axis of the collar body 106 (not shown).

In some instances, the first and second collar sections 102, 104 are configured to interlock to prevent relative movement between the first and second collar sections 102, 104 relative to or along the longitudinal axis. For example, the first collar section 102 includes a pair of elastic hooks 128 that are configured for insertion into corresponding openings in the second collar section 104. The second collar section 104 can include a pair of windows 130 that each accommodate a hook 128 when the sections 102, 104 are connected. In some cases, the snap-fit connection between the first and second collar sections 102, 104 is reversible, i.e., the sections can be separated again by hand. **In** other cases, the snap-fit connection is designed to be permanent.

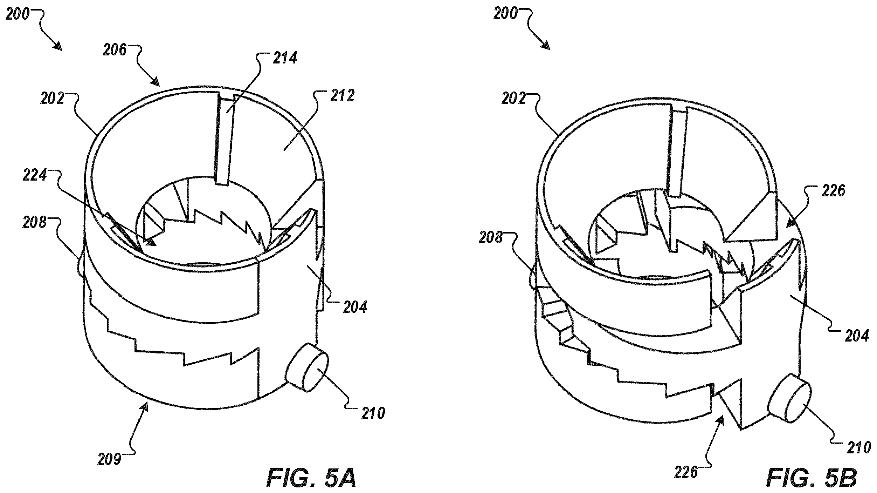

Figures 5A to 5D are perspective views of an example collar 200. Generally speaking, the collar 200 includes a first section 202 and a second section 204 that cooperate to define a collar body 206 configured to engage a needle device. For example, the collar 200 can correspond to the collar 24 shown in Figures 2 and 3 and engage the needle device 10 of Figure 1. The collar body 206 can have a generally cylindrical shape, as shown in Figures 5A and 5B. Similarly to collar 100, an end surface 209 of the collar body 206 can be configured to engage the ridge 18 defined by the tip 16 of the needle device 10, while an angled surface 212 with optional ribs 214 sits against the shoulder of the needle device.

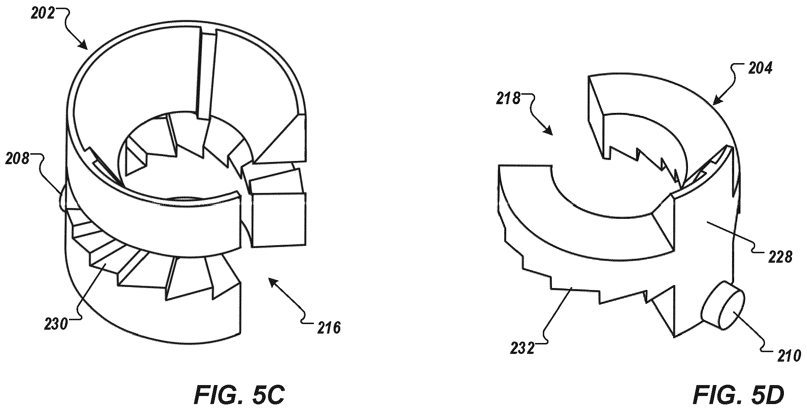

As shown in Figures 5C and 5D, the first collar section 202 and the second collar section 204 each define an opening 216, 218 that extends in parallel to the longitudinal axis of the collar body 206 and is configured to receive the needle device. Unlike the collar sections 102, 104 in Figure 4, the collar sections 202, 204 of the collar 200 extend approximately 270° about the longitudinal axis of the collar body 206. The openings 216, 218 extend approximately 90° and are positioned on opposing sides of the collar body 206, such that the collar sections 202, 204 overlap about the longitudinal axis. Similarly to in collar 100, the collar sections 202, 204 define a smooth cylindrical throughbore 224 (Figure 5A) in the collar body 206 that allows the collar 200 to rotate about the needle device.

Similarly to the previous embodiment, the first and second collar sections 202, 204 are configured to abut along two pairs of mating surfaces 226 shown in Figure 5B. For example, the mating surfaces 226 can extend substantially radially relative to a longitudinal axis of the collar body 206 (not shown). However, unlike the previous embodiment, the two pairs of mating surfaces 226 are not configured to abut at the same time. Specifically, the second collar section 204 comprises an upright section 228 that is received in the opening 216 of the first collar section 202. The upright section 228 is smaller than the opening 216 of the first collar section 202, such that the collar sections 202, 204 can be rotated relative to one another about the longitudinal axis of the collar body. The relative rotation moves the pins 208, 210 of the collar 200, as described in more detail in reference to Figures 6A and 6B.

Figures 6A and 6B schematically depict the collar of Figures 5A to 5D from above. As described above, the first and second collar sections 202, 204 are configured to abut along two pairs of mating surfaces 226A, 226B. In Figure 6A, the first and second collar sections 202, 204 are arranged such that the first pair of mating surfaces 226A abut one another, while the second pair of mating surfaces 226B are separated. In this position, the pins 208, 210 are arranged at an angle relative to one another. In Figure 6B, the positions are reversed, i.e., the first pair of mating surfaces 226A are spaced apart, while the second pair of mating surfaces 226B touch. In the second position, the pins 208, 210 are arranged opposite from one another.

Referring again to Figures 5A to 5D, the first and second collar sections 202, 204 form a ratchet that allows rotation about the longitudinal axis in only one direction (e.g., Figure 6A to 6B but not the other way around). The first and the second collar sections 202, 204 can include teeth 230, 232 that extend in parallel to the longitudinal axis and mate to form the ratchet. Although the collar 200 of Figures 5 and 6 include mating teeth 230, 232, other embodiments may include differently designed ratchets, e.g., a pair of ramps. In addition to the ratchet, the collar sections 202, 204 may include an additional snap-fit connection that allows the collar sections 202, 204 to interlock, e.g., in the position shown in Figure 6B.

Figures 6C to 6E depict an example cap assembly 300 that includes the collar 200 of Figures 5A to 6B. The cap assembly 300 also includes a sleeve 302 and a spring 304. Overall, the sleeve 302 is similar to the sleeve 22. However, the sleeve 302 includes a slot 306 that includes straight portion 308 that extends in parallel to the longitudinal axis and a branched portion 310 that extends laterally to the longitudinal axis. Although not shown in Figures 6C to 6E, the other side of the sleeve 302 includes a straight slot that is similar to slot 30 in Figures 3A and 3B. The pin 208 of the first collar section 202 is arranged in the straight slot (not shown), while the pin 210 of the second collar section 204 is arranged in slot 306.

Initially, the collar 202 is arranged in the position depicted in Figure 6A, and the cap assembly 300 is arranged in the position shown in Figure 6C. As the spring 304 is compressed, the pins travel towards an end surface 312 of the sleeve. The pin 210 approaches a ramp 314 that connects the straight portion 308 and the branched portion 310 that applies a rotational force to the pin 210. The rotational force transitions the collar 200 from the state shown in Figure 6A to the state shown in Figure 6B. The resulting position of the pins 208, 210 allows the pin 210 to travel along the straight portion 308 of the slot 306 (Figure 6D). Due to the ratchet mechanism, the pins 208, 210 are prevented from returning to the state shown in Figure 6A. Accordingly, as the spring 304 relaxes, the pin 210 is forced along the straight portion 308 of the slot. In the position shown in Figure 6E, the user can see that the ratchet mechanism has been activated, i.e., the assembly 300 has been used. This serves as a visual indicator that prevents reuse of the assembly 300.

Figures 7A to 7C depict an assembly that is similar to the assembly of Figures 6C to 6E. For this reason, the same reference numerals are used as in Figures 6C to 6E. In Figures 7A to 7C, the straight portion 308 of the slot 306 includes a retaining hook 316 that maintains the pin 210 in the position shown in Figure 7C. Thus, in addition to having a visual indication that the assembly 300 has been used, the retaining hook 316 physically prevents the assembly 300 from reuse. Although the retaining hook 316 is only shown in Figures 7A to 7C, the slots shown in other embodiments can be provided with a similar retaining hook in some instances.

Figures 6A and 6B depict two relative positions of the pins 208, 210. In some embodiments, the pins 208, 210 may have a third relative position that corresponds to an offset portion arranged at the end of the straight portion 308 (not shown). Once the pins 208, 210 have been moved into the third portion, the pin 210 is unable to re-enter the straight portion 308, thus preventing the re-use of the assembly 300. Such an offset portion can be combined with a retaining hook, such as retaining hook 316, in some cases.

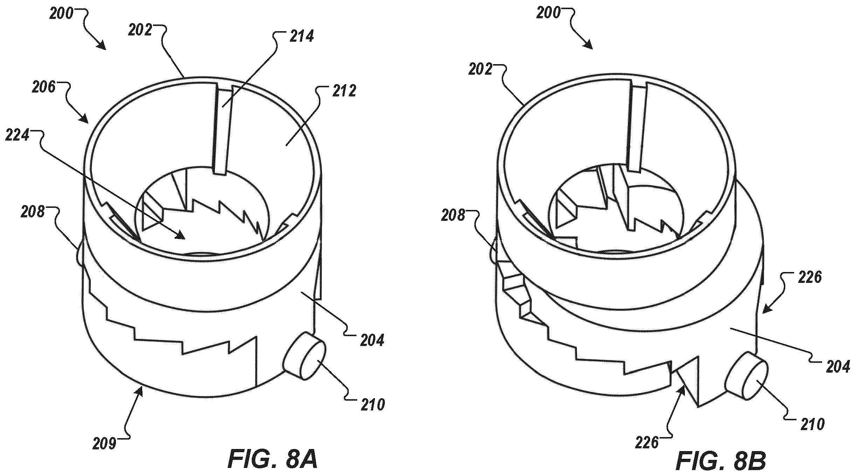

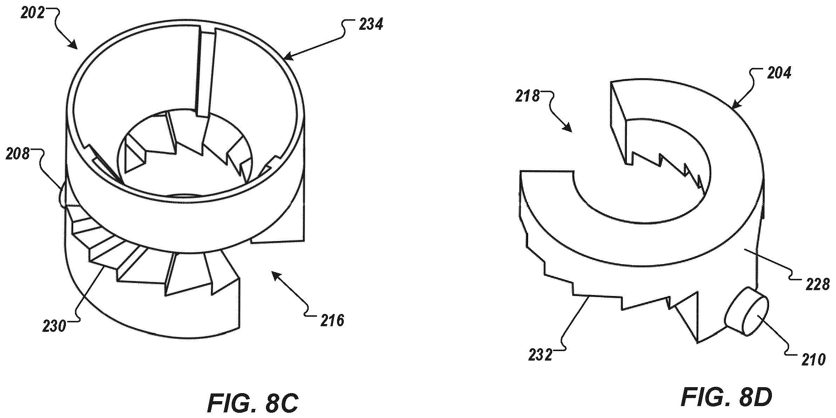

Figures 8A to 8C depict an example collar that is similar to the collar shown in Figures 5A to 5D. For this reason, the same reference numerals are used as in Figures 5A to 5D. As shown in Figures 8C and 8D, the opening 216 in the first collar section 202 does not extend along the entire longitudinal length of the first collar section 202. As a result, the first collar section includes an upper portion 234 that fully encloses the longitudinal axis of the collar body 206. Since the opening 216 does not extend along the entire length of the first collar section 202, the upright section 228 extends along the same portion of the second collar section 204 as the opening 216.

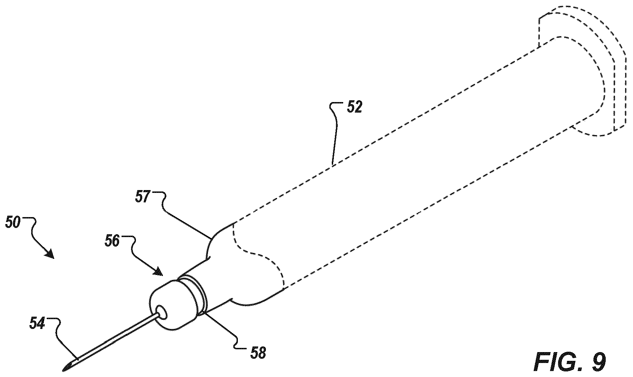

Figure 9 shows an example needle device 50 that is designed to be engaged by the collar 200 shown in Figure 8. The needle device 50 includes a body 52 that is configured to receive a liquid, a hollow needle 54 (e.g., a hypodermic needle) that communicates with an interior of the body 52, and a tip 56 that connects the needle 54 and the body 52. The needle device 50 can include a rounded shoulder 57 that connects the body 52 and the tip 56. The tip 56 of the needle device 50 includes a groove 58 that encircles a longitudinal axis of the needle device 50. The groove 58 is designed, e.g., for snap-fit engagement with the collar body 206 of Figures 8A to 8D. In some cases, other embodiments shown in the other figures can comprise a projection that engages the groove 58.

A number of embodiments have been described. Nevertheless, numerous alternative embodiments within the scope of the claims will be readily appreciated by those skilled in the art. The presently described embodiments are not to be taken as limiting the scope of the invention.

## Claims

1. A cap assembly (20, 300) for enclosing a needle (14) of a needle device (10), the cap assembly comprising:
a sleeve (22, 302) that extends along a longitudinal axis and defines
a first opening (26) configured to receive the needle device,
a second opening (28) opposite to the first opening and sized such that the needle device's needle can protrude through the second opening, and
a slot (30, 306) that extends substantially along the longitudinal axis; and
a collar (24, 200) arranged in the sleeve and biased away from the second opening of the sleeve, wherein the collar is configured to translate along the longitudinal axis of the sleeve and comprises
a first collar section (202) that defines a pin (208) that protrudes through the sleeve's slot, and
a second collar section (204), wherein the first and second collar section cooperate to define a collar body (206) configured to engage the needle device;
wherein the first collar section and the second collar section are configured to rotate relative to one another about the longitudinal axis; **characterized in that**
the first and the second collar sections form a ratchet that allows rotation about the longitudinal axis in only one direction.

2. The cap assembly according to claim 1, wherein the first collar section and the second collar section each define an opening (216, 218) that extends in parallel to the longitudinal axis and is configured to receive the needle device.

3. The cap assembly according to claim 1 or 2, wherein the first collar section and the second collar section are configured to abut along two or more surfaces (226) that extend substantially radially to and along the longitudinal axis.

4. The cap assembly according to any one of the preceding claims, wherein the first collar section and the second collar section are configured to interlock to prevent relative movement of the first and second collar sections along or relative to the longitudinal axis.

5. The cap assembly according to claim 4, wherein the first collar section and the second collar section are configured for snap-fit engagement.

6. The cap assembly according to claim 1, wherein the first collar section encloses the longitudinal axis of the sleeve.

7. The cap assembly according to any one of the preceding claims, wherein the slot comprises a first closed end adjacent to the first opening and a second closed end adjacent to the second opening, and wherein the first closed end comprises a retaining hook (316) configured to engage the pin of the first collar section.

8. The cap assembly according to claim 1, wherein the first and the second collar sections each comprise a plurality of teeth (230, 232) that extend in parallel to the longitudinal axis and mate to form the ratchet.

9. The cap assembly according to claim 1 or 8, wherein the second collar section defines a pin (210) that protrudes through a further slot defined by the sleeve and extending substantially along the longitudinal axis.

10. The cap assembly according to claim 9, wherein the slot that receives the pin of the first collar section extends in parallel to the longitudinal axis, and the slot that receives the pin of the second collar section comprises a straight portion that extends in parallel to the longitudinal axis and a branched portion that extends laterally to the longitudinal axis.

11. The cap assembly according to any one of the preceding claims, wherein the collar consists of the first collar section and the second collar section.

12. An assembly comprising:
the cap assembly according to any one of the preceding claims; and
a needle device that comprises
a body (12) configured to receive a liquid,
a hollow needle (14) that communicates with an interior of the body, and
a tip (16) that connects the needle and the body and defines a portion having an increased diameter,
wherein the collar body comprises an end surface (34) configured to abut the increased diameter portion of the tip of the needle device.

13. An assembly comprising:
the cap assembly according to any one of claims 1 to 11; and
a needle device (50) that comprises
a body (52) configured to receive a liquid,
a hollow needle (54) that communicates with an interior of the body, and
a tip (56) that connects the needle and the body and defines a groove (58) that encircles the longitudinal axis,
wherein the collar body comprises a projection received in the groove of the needle device's tip.

14. The assembly of claim 12 or 13, wherein the needle device is a syringe, an auto-injector, or a pen injector.

## Patentansprüche

1. Kappenbaugruppe (20, 300) zum Umschließen einer Nadel (14) einer Nadelvorrichtung (10), wobei die Kappenbaugruppe Folgendes umfasst:
eine Hülse (22, 302), die sich entlang einer Längsachse erstreckt und
eine erste Öffnung (26) definiert, die zur Aufnahme der Nadelvorrichtung ausgelegt ist,
eine zweite Öffnung (28), die der ersten Öffnung gegenüberliegt und so bemessen ist, dass die Nadel der Nadelvorrichtung durch die zweite Öffnung vorstehen kann, und
einen Schlitz (30, 306), die sich im Wesentlichen entlang der Längsachse erstreckt, und
einen Bund (24, 200), der in der Hülse angeordnet und von der zweiten Öffnung der Hülse weg vorgespannt ist, wobei der Bund zur Translation entlang der Längsachse der Hülse ausgelegt ist und
einen ersten Bundabschnitt (202), der einen Stift (208) definiert, der durch den Schlitz der Hülse vorsteht, und einen zweiten Bundabschnitt (204) umfasst, wobei der erste und der zweite Bundabschnitt zusammenwirken, um einen Bundkörper (206) zu definieren, der zu der Ineingriffnahme der Nadelvorrichtung ausgelegt ist,
wobei der erste Bundabschnitt und der zweite Bundabschnitt dazu ausgelegt sind, sich relativ zueinander um die Längsachse zu drehen, **dadurch gekennzeichnet, dass**
der erste und der zweite Bundabschnitt eine Ratsche bilden, die eine Drehung um die Längsachse in nur einer Richtung gestattet.

2. Kappenbaugruppe nach Anspruch 1, wobei der erste Bundabschnitt und der zweite Bundabschnitt jeweils eine Öffnung (216, 218) definieren, die sich parallel zu der Längsachse erstreckt und zur Aufnahme der Nadelvorrichtung ausgelegt ist.

3. Kappenbaugruppe nach Anspruch 1 oder 2, wobei der erste Bundabschnitt und der zweite Bundabschnitt dazu ausgelegt sind, entlang zwei oder mehr Flächen (226) anzuliegen, die sich im Wesentlichen radial zu und entlang der Längsachse erstrecken.

4. Kappenbaugruppe nach einem der vorhergehenden Ansprüche, wobei der erste Bundabschnitt und der zweite Bundabschnitt dazu ausgelegt sind, sich zu verriegeln, um eine relative Bewegung des ersten und des zweiten Bundabschnitts entlang oder relativ zu der Längsachse zu verhindern.

5. Kappenbaugruppe nach Anspruch 4, wobei der erste Bundabschnitt und der zweite Bundabschnitt für einen Einrasteingriff ausgelegt sind.

6. Kappenbaugruppe nach Anspruch 1, wobei der erste Bundabschnitt die Längsachse der Hülse umschließt.

7. Kappenbaugruppe nach einem der vorhergehenden Ansprüche, wobei der Schlitz ein der ersten Öffnung benachbartes erstes geschlossenes Ende und ein der zweiten Öffnung benachbartes zweites geschlossenes Ende umfasst und wobei das erste geschlossene Ende einen Haltehaken (316) umfasst, der zur Ineingriffnahme des Stift des ersten Bundabschnitts ausgelegt ist.

8. Kappenbaugruppe nach Anspruch 1, wobei der erste und der zweite Bundabschnitt jeweils eine Vielzahl von Zähnen (230, 232) umfassen, die sich parallel zu der Längsachse erstrecken und zur Bildung der Ratsche miteinander koppeln.

9. Kappenbaugruppe nach Anspruch 1 oder 8, wobei der zweite Bundabschnitt einen Stift (210) definiert, der durch einen weiteren Schlitz vorsteht, der durch die Hülse definiert ist und sich im Wesentlichen entlang der Längsachse erstreckt.

10. Kappenbaugruppe nach Anspruch 9, wobei sich der den Stift des ersten Bundabschnitts aufnehmende Schlitz parallel zu der Längsachse erstreckt und der den Stift des zweiten Bundabschnitts aufnehmende Schlitz einen geraden Abschnitt, der sich parallel zu der Längsachse erstreckt, und einen verzweigten Abschnitt, der sich seitlich zu der Längsachse erstreckt, umfasst.

11. Kappenbaugruppe nach einem der vorhergehenden Ansprüche, wobei der Bund aus dem ersten Bundabschnitt und dem zweiten Bundabschnitt besteht.

12. Baugruppe, umfassend:
die Kappenbaugruppe nach einem der vorhergehenden Ansprüche und
eine Nadelvorrichtung, die Folgendes umfasst:
einen Körper (12), der zur Aufnahme einer Flüssigkeit ausgelegt ist,
eine Hohlnadel (14), die mit einem Inneren des Körpers kommuniziert, und
eine Spitze (16), die die Nadel und den Körper verbindet und einen Abschnitt mit einem vergrößerten Durchmesser definiert,
wobei der Bundkörper eine Endfläche (34) umfasst, die dazu ausgelegt ist, an dem Abschnitt mit vergrößertem Durchmesser der Spitze der Nadelvorrichtung anzuliegen.

13. Baugruppe, umfassend:
die Kappenbaugruppe nach einem der Ansprüche 1 bis 11 und eine Nadelvorrichtung (50), die Folgendes umfasst:
einen Körper (52), der zur Aufnahme einer Flüssigkeit ausgelegt ist,
eine Hohlnadel (54), die mit einem Inneren des Körpers kommuniziert, und
eine Spitze (56), die die Nadel und den Körper verbindet und eine Nut (58) definiert, die die Längsachse umgibt,
wobei der Bundkörper einen Vorsprung umfasst, der in der Nut der Spitze der Nadelvorrichtung aufgenommen ist.

14. Baugruppe nach Anspruch 12 oder 13, wobei die Nadelvorrichtung eine Spritze, ein Autoinjektor oder ein Pen-Injektor ist.

## Revendications

1. Ensemble capuchon (20, 300) pour enfermer une aiguille (14) d'un dispositif à aiguille (10), l'ensemble capuchon comprenant :
un manchon (22, 302) qui s'étend le long d'un axe longitudinal et définit
une première ouverture (26) configurée pour recevoir le dispositif à aiguille,
une seconde ouverture (28) opposée à la première ouverture et dimensionnée de telle sorte que l'aiguille du dispositif à aiguille puisse faire saillie à travers la seconde ouverture, et
une fente (30, 306) qui s'étend sensiblement le long de l'axe longitudinal ; et
un collier (24, 200) agencé dans le manchon et sollicité à l'écart de la seconde ouverture du manchon, le collier étant configuré pour se déplacer en translation le long de l'axe longitudinal du manchon et comprenant
une première section de collier (202) qui définit une broche (208) qui fait saillie à travers la fente du manchon, et
une seconde section de collier (204), les première et seconde sections de collier coopérant pour définir un corps de collier (206) configuré pour venir en prise avec le dispositif à aiguille ;
la première section de collier et la seconde section de collier étant configurées pour tourner l'une par rapport à l'autre autour de l'axe longitudinal ; **caractérisé en ce que**
les première et seconde sections de collier forment un cliquet qui permet la rotation autour de l'axe longitudinal dans une seule direction.

2. Ensemble capuchon selon la revendication 1, la première section de collier et la seconde section de collier définissant chacune une ouverture (216, 218) qui s'étend parallèlement à l'axe longitudinal et est configurée pour recevoir le dispositif à aiguille.

3. Ensemble capuchon selon la revendication 1 ou 2, la première section de collier et la seconde section de collier étant configurées pour venir en butée le long de deux surfaces (226) ou plus qui s'étendent sensiblement radialement vers et le long de l'axe longitudinal.

4. Ensemble capuchon selon l'une quelconque des revendications précédentes, la première section de collier et la seconde section de collier étant configurées pour s'interverrouiller afin d'empêcher un mouvement relatif des première et seconde sections de collier le long de l'axe longitudinal ou par rapport à celui-ci.

5. Ensemble capuchon selon la revendication 4, la première section de collier et la seconde section de collier étant configurées pour un engagement par encliquetage.

6. Ensemble capuchon selon la revendication 1, la première section de collier enfermant l'axe longitudinal du manchon.

7. Ensemble capuchon selon l'une quelconque des revendications précédentes, la fente comprenant une première extrémité fermée adjacente à la première ouverture et une seconde extrémité fermée adjacente à la seconde ouverture, et la première extrémité fermée comprenant un crochet de retenue (316) configuré pour engager la broche de la première section de collier.

8. Ensemble capuchon selon la revendication 1, les première et seconde sections de collier comprenant chacune une pluralité de dents (230, 232) qui s'étendent parallèlement à l'axe longitudinal et s'accouplent pour former le cliquet.

9. Ensemble capuchon selon la revendication 1 ou 8, la seconde section de collier définissant une broche (210) qui fait saillie à travers une fente supplémentaire définie par le manchon et s'étendant sensiblement le long de l'axe longitudinal.

10. Ensemble capuchon selon la revendication 9, la fente qui reçoit la broche de la première section de collier s'étendant parallèlement à l'axe longitudinal, et la fente qui reçoit la broche de la seconde section de collier comprenant une partie droite qui s'étend parallèlement à l'axe longitudinal et une partie ramifiée qui s'étend latéralement à l'axe longitudinal.

11. Ensemble capuchon selon l'une quelconque des revendications précédentes, le collier étant constitué de la première section de collier et de la seconde section de collier.

12. Ensemble comprenant :
l'ensemble capuchon selon l'une quelconque des revendications précédentes ; et
un dispositif à aiguille qui comprend
un corps (12) configuré pour recevoir un liquide,
une aiguille creuse (14) qui communique avec l'intérieur du corps, et
une pointe (16) qui relie l'aiguille et le corps et définit une partie ayant un diamètre accru,
le corps de collier comprenant une surface d'extrémité (34) configurée pour venir buter contre la partie de diamètre accru de la pointe du dispositif à aiguille.

13. Ensemble comprenant :
l'ensemble capuchon selon l'une quelconque des revendications 1 à 11 ; et
un dispositif à aiguille (50) qui comprend
un corps (52) configuré pour recevoir un liquide,
une aiguille creuse (54) qui communique avec l'intérieur du corps, et
une pointe (56) qui relie l'aiguille et le corps et définit une rainure (58) qui entoure l'axe longitudinal,
le corps de collier comprenant une saillie reçue dans la rainure de la pointe du dispositif à aiguille.

14. Ensemble selon la revendication 12 ou 13, le dispositif à aiguille étant une seringue, un auto-injecteur ou un injecteur à stylo.
